# EUROPEAN PATENT SPECIFICATION

(11) **EP 0 307 858 B1**
(45) Date of publication and mention of the grant of the patent: **10.03.1993**
(21) Application number: 88114945.4
(22) Date of filing: 13.09.1988
(51) Int. Cl.: G01N 33/564, G01N 33/541, G01N 33/531, G01N 33/535, C12N 15/00

(54) **The SM-B/B1 antigens, the cloning of the SM-B/B1 antigens and the detection of systemic lupus erythematosus by using the SM-B/B1 antigens**
Die SM-B/B1-Antigene, das Klonen der SM-B/B1-Antigene und der Nachweis von systemischem Lupus erythematosus unter Verwendung der SM B/B1-Antigene
Les antigènes SM-B/B1, le clonage des antigènes SM-B/B1 et la détection du lupus erythematosus systémique en utilisant les antigènes SM-B/B1

(30) Priority: 15.09.1987 US 97159
(43) Date of publication of application: 22.03.1989
(73) Proprietor: THE AGOURON INSTITUTE, La Jolla California 92037 (US)
(72) Inventor: Hoch, Sallie O., La Jolla, CA 92037 (US); Rokeach, Luis A., San Diego, CA 92115 (US); Haselby, Jeanne A., Escondido, CA 92026 (US)
(74) Representative: Grünecker, Kinkeldey, Stockmair & Schwanhäusser Anwaltssozietät

(56) References cited:
- EP-A- 0 295 719
- CA-A- 1 183 471
- CHEMICAL ABSTRACTS, vol. 105, no. 9, September 1, 1986, Columbus, Ohio, US; D.G. WILLIAMS et al. "Murine lupus monoclonal antibodies define five epitopes on two different Sm polypeptides." p. 502, abstract no. 77 224v
- CHEMICAL ABSTRACTS, vol. 99, no. 11, September 12, 1983, Columbus, Ohio, US; M.J. FRITZLER et al. "The purification, characterization and amino acid analysis of nuclear ribonuclear protein and Sm antigens reacting with human autoimmune sera." p. 414, abstract no. 86 253p
- CHEMICAL ABSTRACTS, vol. 99, no. 3, July 18, 1983, Columbus, Ohio, US; H. DANG et al. "Molecular and antigenic nature of isolated Sm." p. 473, abstract no. 20 725n
- CHEMICAL ABSTRACTS, vol. 99, no. 11, September 12, 1983, Columbus, Ohio, US; M. ISHAQ et al. "Purification and identification of antignenic polypeptides of Sm and RNP antigens of goat liver." p. 418, abstract no. 86 285a
- CHEMICAL ABSTRACTS, vol. 97, no. 25, December 20, 1982, Columbus, Ohio, US; W.H. SCHRIER et al. "Identification of an antigenic protein recognized by anti-Sm autoantibody." p. 672, abstract no. 214 055y
- ARTHRITIS AND THEUMATISM, vol. 29 (1986), pp. 986-996
- J. BIOL. CHEM., vol. 259 (1984), pp. 12850-12856
- J. BIOL. CHEM., vol. 259 (1984), pp. 5907-5914

## Description

This invention relates to a recombinant polypeptide capable of reacting with an anti-Sm antibody, a DNA fragment coding for said polypeptide, a vector comprising said DNA fragment, a method of producing the recombinant polypeptide and a method of testing a serum sample of a living being to detect whether or not the living being is affected by systemic lupus erythematosus.

In an autoimmune disease, the body's immune system generates antibodies which react with the body's own cellular components. The resulting formation of immune complexes can lead to a variety of pathological conditions. Some rheumatic diseases are autoimmune diseases. One type of such rheumatic disease is systemic lupus erythematosus.

Antinuclear antibodies are a hallmark of autoimmune diseases. The antibodies are antinuclear because they act against components in the nucleus of the cells in the living beings. Assays based upon the measurement of these antibodies are well established in the clinical laboratory for diagnosis of a variety of rheumatic diseases. (1,2). The assays in general do not employ a purified antigen as the target of these antibodies. Thus, the assays are relatively crude. This results from the fact that cellular extracts routinely serve as the source of the antigen so that there is no defined standard to serve as an internal control. The problem has been complicated because the molecular nature of the target antigens for these autoimmune antibodies is relatively complex.

In recent years, persons skilled in the art have begun to decipher the molecular nature of the target antigens for the autoimmune antibodies and, in particular, to recognize their complexity relative to the large structural elements of which they may be a part. The large structural element may be specifically ribonucleoprotein (RNP) or deoxyribonucleoprotein (DNP) particles. Persons skilled in the art have also begun to recognize the complexity of these antigens relative to their roles in the functioning of the cells in living beings. (3). With the advent of such information, the potential exists for the development of defined and abundant antigen reagents and for the use of these reagents in the development of sensitive and quantitative clinical assays.

Attempts have been made to isolate relatively simple polypeptides or proteins as the antigens in detecting the antibodies in autoimmune diseases such as rheumatic diseases. Although these polypeptides or proteins are simple antigens, they are actually part of yet more complex cellular structures. As a result, it has been difficult to isolate the polypeptides or proteins which react with such antibodies. It has also been difficult to develop an assay in which such antigens are used to detect the antibodies of the autoimmune diseases such as rheumatic diseases.

It has been particularly difficult to develop a defined and abundant antigen for the rheumatic disease designated as systemic lupus erythematosus since the antigen (designated Sm) for systemic lupus erythematosus is associated with a conserved class of proteins called the snRNP (small nuclear ribonucleoproteins) (4). The Sm small nuclear ribonucleoproteins (snRNP) associated with the Sm antigens contain five species of U snRNA (U1, U2, U4, U5 and U6) and at least eleven polypeptides. The U snRNA are rich in uridine.

Applicants and other persons skilled in the art have identified Sm snRNP. (5-8). Applicants have recently isolated the Sm-D polypeptide antigen associated with systemic lupus erythematosus. The Sm-D polypeptide has a molecular weight of approximately thirteen thousand (13,000). Applicants have also recently formulated a simple and effective colorimetric assay in which such antigen can be used with a serum sample of a living being to identify whether the living being is affected by systemic lupus erythematosus. All of the above is disclosed and claimed in European patent application 88109795.0 (EP-A-0295719) for "The Sm-D Antigen, The Cloning of the Sm-D Antigen and The Detection of Systemic Lupus Erythematosus By Using the Sm-D Antigen" and assigned or record to the assignee of record in this application. The European patent application forms part of the prior art under Article 54(3) EPC.

In Arthritis and Rheumatism, Volume 29 (1986), pages 986 to 996, the analysis of sera of patients with mixed connective tissue diseases and systemic lupus erythmatosus is reported. The sera are analyzed for the existance of auto-antibodies by using the technique of immuno blotting. However, this document does not disclose any amino acid sequence information about the detected antigens.

In J. Biol. Chem., Volume 259 (1984), pages 12850 to 12856, several polypeptides are characterized by their molecular weight and their reactivity with human anti-Sm auto antibodies. However, this document does not provide any amino acid sequence information about the several detected proteins. Moreover, this document leaves open as to whether the resolved protein bands represent a single molecular species.

Furthermore, J. Biol. Chem., Volume 259 (1984), pages 5907 to 5914, describes the molecular weights of mammlian small nuclear ribonucleoproteins which are detectable with anti-Sm sera and a mouse monoclonal antibody, respectively. Particular amino acid sequences are also not disclosed in this reference and the purity of the observed bands remains obscure.

The object of the present invention is to provide a recombinant polypeptide which is reactive with an anti-Sm antibody.

This object is achieved by a recombinant polypeptide comprising the following sequence:
val gly lys ser ser lys met leu gln his ile asp tyr arg met arg cys ile leu gln asp
and said recombinant polypeptide comprises at least one epitope capable of reacting with an anti-Sm antibody.

A further object is to provide a DNA fragment, a vector and a transformed host which are useful for preparing the above polypeptide.

These objects are achieved by a DNA fragment comprising a nucleotide sequence coding for the above recombinant polypeptide, a vector comprising such a DNA fragment and a host comprising the DNA fragment or the vector.

A further object is to provide a method of producing the above recombinant polypeptide.

This object is achieved by a method comprising the steps of
(a) isolating a naturally-occurring polypeptide that is associated with systemic lupus erythematosus,
(b) identifying the amino acid sequence of at least a portion of the naturally-occurring polypeptide,
(c) producing a nucleic acid probe based upon the amino acid sequence identified in step (b), wherein the nucleic acid probe is capable of reacting with at least a portion of the DNA that encodes the naturally-occurring polypeptide,
(d) reacting the nucleic acid probe with a DNA library,
(e) isolating the DNA that reacts with the nucleic acid probe, and
(f) using the isolated DNA or a synthetic DNA to produce the recombinant polypeptide, wherein said synthetic DNA is capable of encoding the same polypeptide as the isolated DNA,
wherein said naturally-occurring polypeptide is selected from a group consisting of Sm-B and Sm-B' polypeptides.

This object is also achieved by a method comprising the step of culturing a transformed host comprising a vector coding for the recombinant polypeptide of the invention which host is capable of expressing the foreign DNA.

It is a further object to provide a method of testing a serum sample of a living being to detect whether or not the living being is effected by systemic lupus erythematosus.

This object is achieved by a method which includes the steps of
(a) reacting the recombinant polypeptide of Claim 1 with the serum sample to be tested, and
(b) determining the presence or amount of antibody contained in the sample reactive with the polypeptide.

It is a further object to provide a test kit for the detection of antibodies associated with systemic lupus erythematosus.

This object is achieved by a test kit comprising
(a) the recombinant polypeptide of Claim 1,
(b) an antihuman IgG/IgM antibody that is linked to an enzyme, and
(c) a substrate that is capable of reacting with the enzyme to produce a detectable reaction.

Applicants have now isolated cDNA encoding at least one of the Sm-B and/or Sm-B' polypeptide antigens. The Sm-B and/or Sm-B' polypeptide antigens respectively have molecular weights of twenty six thousand (26,000) and twenty seven thousand (27,000). (5-8). Applicants have also formulated a simple and effective colorimetric assay in which such antigens can be used with a serum sample of a living being to identify whether the living being is affected by systemic lupus erythematosus. Applicants have also formulated a simple and effective colorimetric assay involving a mixture of the Sm-D and Sm-B and/or Sm-B' polypeptide assays.

In one embodiment of the invention, snRNP proteins are isolated, as by immunoaffinity chromatography, using antibodies from a serum sample of a living being affected by systemic lupus erythematosus. The Sm-B and/or Sm-B' polypeptides are in turn isolated from the snRNP protein as by gel electrophoresis and electroelution. The amino termini of the Sm-B and/or Sm-B' polypeptides are then sequenced, and labelled DNA probes with a nucleotide sequence complementary to that encoding the amino acid sequence are synthesized. A human cDNA library in a lambda cloning vector is transferred to appropriate filters such as nitrocellulose filters. These filters are screened as by hybridization with the labelled probes to identify the cDNA clones in the library with sequences matching those of the labelled probes.

The cDNA encoding the Sm-B and/or Sm-B' proteins are then subcloned. The Sm-B and/or Sm-B' polypeptides are then isolated as by immunoaffinity chromatography and, if further desired, as by HPLC chromatography. An assay is then made with the isolated Sm-B and/or Sm-B' polypeptides by reacting the Sm-B and/or Sm-B' polypeptides with a serum sample of a living being to determine whether the living being is affected by systemic lupus erythematosus. The assay may also be made with a mixture of the isolated Sm-B and/or Sm-B' polypeptides and the isolated Sm-D polypeptide (isolated as described above).

The assay may be performed as by an enzyme-linked immunoabsorbant assay such as one using anti-human IgG/IgM antibodies covalently attached to an enzyme indicator. Lactoperoxidase/alkaline phosphatase are each an example of an enzyme indicator to indicate, as by a distinctive color, an affected person.

In the drawings:
Figure 1 indicates the sequence of a number of amino acids at the amino terminus of the Sm-B and/or Sm-B' polypeptides and further indicates probes synthesized to hybridize with the cDNA encoding such amino acid sequence; and

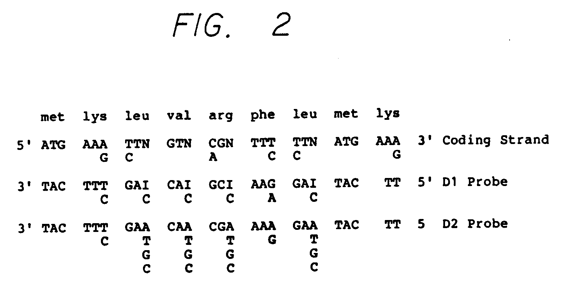

Figure 2 indicates the sequence of a number of amino acids at the amino terminus of the Sm-D polypeptide and further indicates probes synthesized to hybridize with the cDNA encoding such amino acid sequence as disclosed in the European patent application 88109795.0.

### Isolation of Sm SnRNP

In one embodiment of the invention, the Sm snRNP was isolated by immunoaffinity chromatography. As a first step in such process, a plasmapheresis of a patient affected by systemic lupus erythematosus was obtained as the source of human anti-Sm antibodies. The immunoglobulin G (IgG) was purified by half-saturation ammonium sulfate fractionation and passage over a DEAE-Sephacel® column The isolated IgG was coupled to Sepharose® CL-4B by a carbonyl - diimidazole procedure. (10).

Extracts were prepared from suspension cultures of a human cell line designated as HeLa as by sonication of total cells in 0.35M NaCl, 10mM Tris-HCl, pH 7.4, 1.5mM MgCl₂, 0.2mM phenylmethylsulfonyl fluoride. (8). The cell extracts were passed immediately after preparation through the anti-Sm immunoaffinity column which was prepared as discussed in the preceding paragraph. The columns were then washed with an extraction buffer and were eluted in 10mM Tris-HCl, pH 7.4, containing 0.1 MNaCl, 6M urea and 0.2 mM phenylmethylsulfonyl fluoride. Carrier RNA (20µ g/ml) was added to the eluate to facilitate the subsequent precipitation of the Sm snRNP at dilute protein concentrations. The Sm snRNP was then precipitated overnight at -20°C. by the addition of two (2) volumes of ethanol.

The immunoaffinity column eluates were analyzed for total protein content (including the characteristic Sm snRNP profile) by Coomassie blue stain after sodium dodecyl sulfate (SDS) gel electrophoresis (11). Antigenic polypeptides were detected by immunoblot after transfer to nitrocellulose. (12, 13). In this way, the Sm antigens were purified on a bulk basis in sufficient purity for direct biochemical analysis. This procedure minimized protease and nuclease exposure of the Sm snRNP (9).

### Isolation of Individual Sm snRNP Polypeptides

Although applicants were able to isolate the Sm snRNP particle readily by chromatographic techniques, they were not able to fractionate clearly the Sm snRNP particle by chromatographic techniques. Instead, applicants fractionated the Sm snRNP particle by using SDS-polyacrylamide gel electrophoresis and electroelution. The protocol (and apparatus) used by applicants to accomplish this was that used by Hunkapiller et al (14) to isolate microgram quantities of protein. This protocol was ideal for applicants' purposes because, for each polypeptide band, they were able to elute a small amount of protein (10-20 µg) from a large block of acrylamide gel (10-11cm in width/1.5mm in thickness) into a small volume (300-500 µl).

The only modification made by applicants in the Hunkapiller et al protocol was to change the staining conditions prior to excising individual bands across the gel. This change was made because a number of the peptides of interest were quite closely spaced even on long (20 cm) gels. In order to be able to clearly distinguish between such peptides of interest after the usual destaining period of two (2) hours, the Coomassie blue was decreased to five hundredths of one percent (0.05%) from the original protocol of Hunkapiller et al. After electroelution of individual bands of peptides, each band was checked for homogeneity by silver stain after the SDS gel electrophoresis (11) and for immunoreactivity by protein blot. (12, 13).

### Protein Sequencing

Applicants chose a gas-phase system to sequence the amino acids in the Sm-B and/or Sm-B' polypeptide because only pmole amounts of protein were required. (15, 16). The sequencing was performed at the Protein Chemistry Laboratory, Washington University, St. Louis, Missouri. Applicants obtained sequences of nineteen (19) and twenty (20) residues for the Sm-B and/or Sm-B' polypeptides respectively at the amino terminus of the Sm-B and/or Sm-B' polypeptides. The sequence was as follows:
val gly lys ser ser lys met leu gln his ile asp tyr arg met arg cys ile leu gln asp

### Molecular Cloning of the Sm-B and/or Sm-B' proteins cDNAs.

### (a) Design of probes

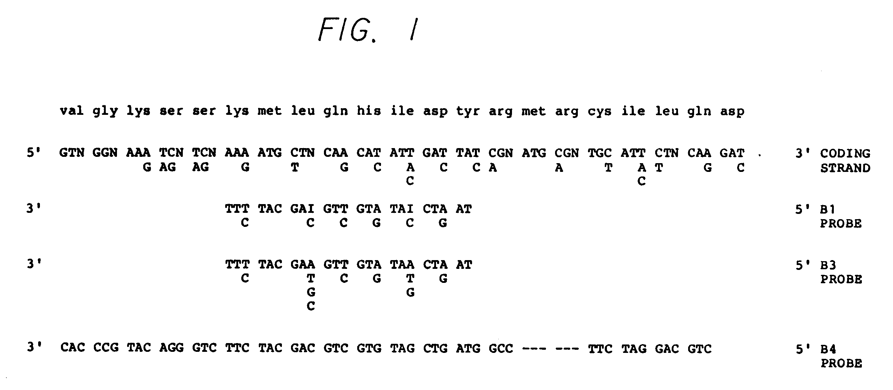

Applicants synthesized two (2) twenty three (23) - mer and one (1) fifty four (54) - mer oligonucleotide probes (ABI Synthesizer, The Agouron Institute) in order to clone the cDNA encoding the Sm-B and/or Sm-B' protein. These probes were synthesized with a base composition deduced from the amino acid sequence available from the sequencing of the Sm-B and/or Sm-B' polypeptide(s). These probes were designated as the B1, B3 and B4 probes. The probes were labelled as by radioactive phosphorus. 
The B1, B3 and B4 probes were formed as follows:

val gly lys ser ser lys met leu gln his ile asp tyr arg met arg cys ile leu gln asp

5' GTN GGN AAA TCN TCN AAA ATG CTN CAA CAT ATT GAT TAT CGN ATG CGN TGC ATT CTN CAA GAT 3' CODING
                 G AG  AG    G     T   G   C   A   C   C   A       A   T   A   T   G   C    STRAND
                                               C                           C

3'               TTT TAC GAI GTT GTA TAI CTA AT 5' B1
                      C   C   C   G   C   G        PROBE

3'               TTT TAC GAA GTT GTA TAA CTA AT 5' B3
                      C   T   C   G   T   G        PROBE
                          G           G
                          C

3' CAC CCG TAC AGG GTC TTC TAC GAC GTC GTG TAG CTG ATG GCC --- --- TTC TAG GAC GTC 5' B4
                                                                                      PROBE
This is also shown in Figure 1. In the above sequences, "N" represents any of the four (4) nucleotides A, T, G and C. "I" substitutes for all of the four (4) nucleotides except the nucleotide "C". This corresponds to deoxyinosine. In the above sequence, cysteine is shown as one of the amino acids. This has been confirmed by subsequent experiments. Actually, however, during the week with the B1, B2 and B4 probes this amino acid was actually thought to be lysine.

As will be seen, the B1 probe was designed to use deoxyinosine at the third position of the most ambiguous codons. (17). The B1 probe was accordingly formed as a mixture of sixty four (64) different oligonucleotides. In the B3 probe, all four (4) nucleotides were included at the third (3rd) position of redundant codons. Furthermore, based upon codon usage (18), applicants considered only cytosine at the first position of leucine triplets. As a result, the total number of molecular species for the B3 probe was reduced to one hundred and ninety two (192). For the B4 probe, a single fifty four (54) - mer species was designed based upon human codon usage (18) for the known amino acids of the Sm-B and/or Sm-B' polypeptides.

### (b) Library Screening

As the source of cDNA, a library containing λgt10 cloning vectors and made from human B lymphocyte poly-RNA was purchased from Clonetech Laboratories, Inc., of Palo Alto, California. Approximately two hundred and fifty thousand (250,000) independent λ recombinant plaques were plated on E. coli Y1090 bacterial lawn and transferred to nitrocellulose filters. (19). These filters were screened by hybridizing them with the [³²P]-labelled B1 probe.

From the first round of screening, nineteen (19) plaques were identified as having become hybridized with the [³²P]-labelled B1 probe. Approximately two hundred and fifty thousand (250,000) additional independent λ recombinant plaques were screened with probe B3 as described above. In this screening, sixteen (16) plaques were identified as having become hybridized with the [³²P]-labelled B3 probe.

The nine (9) strongest positive clones from the screening with the B1 probe and all sixteen (16) positive clones from the screening with the B3 probe were subjected to two (2) more cycles of purification involving the use of the B3 probe. After two (2) such additional cycles of hybridization, a total of twenty-four (24) pure positive clones were obtained. These twenty four (24) clones were further screened with the B4 probe under low salt and high temperature conditions. From this last screening, seven (7) recombinant clones were identified as having become hybridized with the [³²P]-labelled B4 probe.

### (c) Subcloning and Sequencing

DNA from the twenty four (24) lambda positive clones screened with the B3 probe was prepared (19) and digested with EcoRI enzyme. The size of the cDNA inserts was determined after such digestion. The inserts were classified into six (6) groups each containing inserts within a particular range of nucleotide lengths. These groups ranged in lengths from 0.80 Kb to 2.0 Kb. From the seven (7) positive clones identified with the B4 probe, six (6) belonged to the 1.3-1.4 Kb size group. The positive clone λ B419-2 had an insert size of 1.1kb. The cDNA EcoRI inserts from recombinants λ B3-1, λ B22-2, λ B407-1, λ B419-2 and λ B443-2 were subcloned into a generic vector designated as M13mp18 (20). The cDNA EcoRI fragments from clones λ B409-2 and B445-2 were subcloned into a generic vector designated pUC18 (21). The nucleotide sequence of the cDNA inserts was determined by employing the dideoxy method (22). All seven (7) clones were found to contain a DNA sequence perfectly matching the codon composition for the known sequence of twenty (20) amino acids in the Sm-B and/or Sm-B' polypeptides.

### (d) Expression of the Recombinant Sm-B and/or Sm-B' Proteins

Expression of the Sm-B and/or Sm-B' proteins may be provided in E. coli. There are two (2) major reasons for this choice: (1) this bacterium is the most studied and understood organism in terms of its physiology, genetics and molecular biology; and (2) a wide variety of convenient expression vectors already exist. (23-26). These vectors utilize strong bacterial transcriptional and translational signals to achieve high levels of synthesis of the cloned gene product.

To prevent problems of inhibition of bacterial growth by the cloned gene product, expression of the cloned gene product may be conditionally activated by using inducible promoters of transcription. For example, plasmids pIN-III (23) and pKK233-2 (24) respectively contain the lac and tac promoters. Both are inducible by the addition of isopropyl- β - D-thiogalactopyranoside (IPTG) to the cultures. Plasmid pAS1 (25) bears the λpL promoter which is activated by shifting the incubation temperature from 30°C. to 42°C. Another system (26) is based on the use of two (2) plasmids. One plasmid pGP1-2 encodes the T7 RNA polymerase under the control of the λ pL promoter. The gene of interest is cloned in a second plasmid pT7-3 under the control of the φ10 T7 RNA polymerase promoter.

As a first step, the cDNA encoding the Sm-B and/or Sm-B' proteins may be cloned in all of the vectors cited above. Secondly, expression of the recombinant Sm-B and/or Sm-B' protein may be optimized by determining the best incubation conditions before and after the induction of the bacterial transcriptional promoter. Levels of expression of the cloned Sm-B and/or Sm-B' polypeptides can be monitored by labelling the newly synthesized polypeptides with [³⁵S] - methionine and taking at different times samples which are then subjected to SDS - polyacrylamide electrophoresis and autoradiography.

### Assays for anti-Sm antibodies

A direct assay may be used to determine the reactivity of the antibodies in a living being such as a patient. A major limitation to a direct assay is generally that a relatively large quantity of antigen is required to make such an assay. This is particularly true when it is desired to have the antigen at or near complete homogeneity.

Large quantities of antigen may be produced as discussed two (2) paragraphs previously. For example, the Sm-B and/or Sm-B' antigen may be isolated as by immunoaffinity chromatography discussed in the section entitled "Isolation of Sm snRNP". In such immunoaffinity chromatography, a polyclonal human serum may be used. Alternatively, a rabbit polyclonal antibody may be employed which has specificity for the Sm-B and/or Sm-B' bands. If this is considered desirable, chromatography may be employed on the antigen as a final purification step.

An enzyme-linked immunoabsorbent assay (27) is the preferred assay. The purified Sm-B and/or Sm-B' polypeptides are preferably absorbed on the surface of microtiter plates. Any exposed surfaces on the microtiter plates are preferably blocked with a concentrated non-reactive protein solution (e.g. bovine serum albumin) (28a). The serum sample to be tested is preferably allowed to react directly with the Sm-B and/or Sm-B' antigens.

Any antigen-antibody immune complex formed as a result of the reaction of the serum sample with the Sm-B and/or Sm-B' antigen is preferably detected as by anti-human IgG/IgM antibodies covalently attached to an enzyme indicator (e.g. lactoperoxidase/alkaline phosphatase). The quantitation of the attached enzyme indicator may be indicated as by a colorimetric assay. Furthermore, the microtiter plates can be read on an automatic plate reader. Background measurements can also be obtained through appropriate controls by using a serum from an unaffected person to provide a negative response and by using a known human anti-Sm autoantibody (29) or a rabbit anti-Sm-B and/or Sm-B' antibody (28b) to obtain a positive response.

Instead of providing only the Sm-B and/or Sm-B' antigens as described above to test for systemic lupus erythematosus, a mixture of the Sm-D antigen and the Sm-B and/or Sm-B' antigens may be provided or the Sm-D antigen may be mixed only with the Sm-B antigen or the Sm-B' antigen. When the Sm-D antigen is used with one or both of the Sm-B and/or Sm-B' antigens, a mouse anti-Sm-D monoclonal antibody may be mixed with the rabbit anti-Sm-B and/or Sm-B' antibody to obtain a positive response. Alternately, either the Sm-B antigen or the Sm-B' antigen may be individually mixed with the mouse anti-Sm-D monoclonal antibody or the rabbit anti-Sm-B and/or Sm-B' antibody to obtain the positive response.

As disclosed and claimed in European patent application 88109795.0,(EP-A-295719) Sm-D polypeptide was isolated by using D1 and D2 probes as follows: 
met   lys   leu   val   arg   phe   leu   met   lys

5' ATG   AAA   TTN   GTN   CGN   TTT   TTN   ATG   AAA   3' Coding Strand
           G   C           A       C   C             G

3' TAC   TTT   GAI   CAI   GCI   AAG   GAI   TAC    TT   5' D1 Probe
           C     C     C     C     A     C

3' TAC   TTT   GAA   CAA   CGA   AAA   GAA   TAC    TT   5  D2 Probe
           C     T     T     T     G     T
                 G     G     G           G
                 C     C     C           C
A cDNA library in the λgt10 cloning vector was screened by using the D1 and D2 probes. The screening was provided substantially as described above and specifically as described in the above European patent application. DNA selected in accordance with such screening process was subcloned and sequenced as described in the above European patent application.

The recombinant Sm-D protein may be expressed substantially as described above and specifically as described in the European patent application 88109795.0.

The major Sm antigens are associated with the D, B and B' polypeptides. Three (3) references are cited below in which investigators have specifically looked for anti-Sm reactivity by using a technique called the immunoblot. (30-32). The most common pattern is to see all three (3) of these polypeptides. Furthermore, it is known that these three (3) polypeptides share at least one (1) epitope in common because there is a mouse monoclonal antibody which recognizes all three (3) of the polypeptides. (33). However, there are also unique determinants. In the three (3) surveys cited (30-32), approximately eight percent (8%) of the human antisera recognized only the Sm-B and/or Sm-B' polypeptide. Presumably there are also rare sera that recognize only the Sm-D polypeptide. For example, a mouse monoclonal antibody has been isolated which recognized only the Sm-D polypeptide. (34).

Researchers working in this field have tended to emphasize the Sm-D antigen thus far. The clinical source of tissue for anti-Sm assays is very frequently an extract called ENA which is made from rabbit thymus. It has been shown that, in the classic way in which ENA is prepared, most, if not all, of the Sm-B and/or Sm-B' polypeptides were being lost. (35). Thus, it could be concluded that in many clinical assays anti-Sm reactivity was directed only against the Sm-D polypeptide. Moreover, as concluded in reference 31, "the presence of anti-D antibodies seems more characteristic for anti-Sm sera since they are not found in anti-(U1) RNP sera". Stated differently, anti-Sm-B and/or Sm-B' antibodies may be associated with more than one (1) phenotype.

All three antigens (Sm-D and Sm-B and/or Sm-B') appear to be important. The Sm-D antigen appears to be the most important of the three (3) but the Sm-B and/or Sm-B' antigens also appear to provide a significant contribution. Because of this, a mixture of the three (3) antigens appears to be more desirable, at least in certain instances, than any individual one of these antigens.

The methods described above have certain important advantages. They provide for the cloning of the Sm-B and/or Sm-B' antigens to detect systemic lupus erythematosus. They also provide for the reaction of these antigens with a serum sample of a person to detect whether the person is affected by systemic lupus erythematosus. Another antibody with an enzyme can be included to produce a distinctive color when the antibody of systemic lupus erythematosus reacts with the Sm-B and/or Sm-B' antigens (or with the mixture of the Sm-D and Sm-B and/or Sm-B' antigens) to produce an antigen-antibody complex, and the other enzyme-linked antibody reacts with the antigen-antibody complex and is detected by a colorimetric assay for activity of the enzyme. This distinctive color can be detected to indicate that the person being tested is affected by systemic lupus erythematosus.

The methods described above also have other important advantages. Since the antigens are only the Sm-B and/or Sm-B' polypeptides (alone or mixed with the Sm-D antigen) rather than a complex containing the Sm-D polypeptide and a number of other interfering polypeptides and proteins, the reaction of the Sm-B and/or Sm-B' polypeptides (or the mixture of these polypeptides with the Sm-D polypeptide) with the antibodies of systemic lupus erythematosus is more specific and sensitive than the reaction of such complexes with such antibodies as in the prior art. This causes applicants' method of detection to be more definite and certain than the tests on the complexes of the prior art.

### LITERATURE CITED

1. Fritzler, M.J. 1986. Methods Achiev. Exp. Pathol. 12:224.
2. McCarty, G.A. 1986. Med. Clin. North Am. 70:237.
3. Brunel, C., J. Sri-Widada and P. Jeanteur. 1985. Prog. Mol. Subcell. Biol. 9:1.
4. Lerner, M.R. and J.R. Steitz. 1979. Proc. Natl. Acad. Sci. U.S.A. 76:5495.
5. Hinterberger, M., I. Pettersson, and J.A. Steitz. 1983. J. Biol. Chem. 258:2604.
6. Kinlaw, C.S., B.L. Robberson, and S.M. Berget. 1983. J. Biol. Chem. 258:7181.
7. Bringmann, P., J. Rinke, B. Appel, R. Reuter, and R. Luhrmann. 1983. EMBO J. 2:1129.
8. Billings, P.B. and S.O. Hoch. 1980. J. Biol Chem. 259:12850.
9. Billings, P.B. and S.O. Hoch. 1983. J. Immunol. 131:347-351.
10. Hearn, M.T.W., E. L. Harris, G.S. Bethell, W.S. Hancock and J.A. Ayers. 1981. J. Chromatogr. 218:509.
11. Laemmli, U.K. 1970. Nature (Lond.) 227:680.
12. Billings, P.S., J.R. Barton and S.O. Hoch. 1985. J. Immunol. 135:428.
13. Towbin, H., T. Staehelin and J. Gordon. 1979. Proc. Natl. Acad. Sci. U.S.A. 78:4350.
14. Hunkapiller, M.W., E. Lujan, F. Ostrander and L.E. Hood. 1983. Methods Enzymol. 91:277.
15. Hewick, R.M., M.W. Hunkapiller, L.E. Hood and W.J. Dreyer. 1981. J. Biol. Chem. 256:7990.
16. Hunkapiller, M.W., R.M. Hewick, W.J. Dryer and L.E. Hood. 1983. Methods Enzymol. 91:399.
17. Ohtsuka, E., S. Matsuki, M. Ikebasa, Y. Takahashi and K. Matsubana. 1985. J. Biol. Chem. 260:2605.
18. Lathe, R. 1985. J. Mol. Biol. 183:1.
19. Maniatis, T., E.F. Fritsch and J. Sanbrook, 1982. Molecular Cloning: A Laboratory Manual. Cold Spring Harbor Laboratory Press, Cold Spring Harbor, NY.
20. Norrander, J., T. Kempe, and J. Messing. 1983. Gene. 26:101.
21. Yanish-Perron, C., J. Vierra, and J. Messing. 1985. Gene. 33:103.
22. Sanger, F., S. Nickler and A.R. Coulson. 1977. Proc. Natl. Acad. Sci. U.S.A. 74:5463.
23. Masui, Y., J. Coleman, and M. Inouye. 1983. In Experimental Manipulation of Gene Expression. Academic Press, Inc. New York, NY pp.15.
24. Amann, E., and J. Brosius. 1987. Gene. In press.
25. Shatzman, A., Y-S Ho, and M. Rosenberg. 1983. In Experimental Manipulation of Gene Expression. Academic Press, Inc. New York, NY. pp.1.
26. Tabor, S. and C.C. Richardson. 1985. Proc. Natl. Acad. Sci. U.S.A. 82:1074.
27. Engvall, E. and P. Perlmann. 1971. Immunochemistry 8:871.
28a. Billings, P.B., R.W. Allen, F.C. Jensen and S.O. Hoch. 1982. J. Immunol. 128:1176.
28b. S.O. Hoch, unpublished.
29. Tan, E.M., M.J. Fritzler, J.S. McDougal, F.C. McDuffie, R.M. Nakamura, M. Reichlin, C.B. Reimer, G.C. Sharp, P.H. Schur, M.R. Wilson and R.J. Winchester. 1982. Arthritis Rheum. 25:1003.
30. Pettersson, I., M. Hinterberger, T. Mimora, E. Gottlieb and J. Stietz. 1984. J. Biol. Chem. 259:5907.
31. Habets, W.J.J.H.M Berden, S.O. Hoch and W.J. van Venrooij. 1985. Eur. J. Immunol. 15:992.
32. Pettersson, I., G. Wang, E.I. Smith, H. Wigzell, E. Hedfors, J. Horn and G.C. Sharp. 1986. Arthritis Rheum. 29:986.
33. Lerner, E.A., M.R. Lerner, C.A. Janeway and J.A. Stietz. 1981. Proc. Natl. Acad. Sci. USA 78:2737.
34. Billings, P.B., J. Barton and S.O. Hoch. 1985. J. Immunol. 135:428.
35. Billings, P.B. and S.O. Hoch. 1983. J. Immunol. 131:347.

## Claims

1. A recombinant polypeptide comprising the following sequence:
val gly lys ser ser lys met leu gln his ile asp tyr arg met arg cys ile leu gln asp
wherein said recombinant polypeptide comprises at least one epitope capable of reacting with an anti-Sm antibody.

2. A DNA fragment comprising a nucleotide sequence coding for the recombinant polypeptide of Claim 1.

3. The DNA fragment of Claim 2 further comprising a nucleotide sequence capable of influencing the expression of the sequence coding for the recombinant polypeptide.

4. The DNA fragment of Claim 3, wherein said further nucleotide sequence encodes a promoter.

5. The DNA fragment of any of Claims 2 to 4, wherein said fragment is capable of expressing in a host cell the polypeptide of Claim 1.

6. A vector comprising the DNA fragment of any of Claims 2 to 5.

7. The vector of Claim 6, wherein said vector is capable of transforming a host cell.

8. A transformed host comprising the DNA fragment of any of Claims 2 to 5.

9. A transformed host comprising the vector of Claim 7.

10. The transformed host of Claim 9, wherein said host is capable of expressing said DNA fragment contained in said vector.

11. A method of producing the recombinant polypeptide of Claim 1 comprising the steps of
(a) isolating a naturally-occurring polypeptide that is associated with systemic lupus erythematosus,
(b) identifying the amino acid sequence of at least a portion of the naturally-occurring polypeptide,
(c) producing a nucleic acid probe based upon the amino acid sequence identified in step (b), wherein the nucleic acid probe is capable of reacting with at least a portion of the DNA that encodes the naturally-occurring polypeptide,
(d) reacting the nucleic acid probe with a DNA library,
(e) isolating the DNA that reacts with the nucleic acid probe, and
(f) using the isolated DNA or a synthetic DNA to produce the recombinant polypeptide, wherein said synthetic DNA is capable of encoding the same polypeptide as the isolated DNA,
wherein said naturally-occurring polypeptide is selected from a group consisting of Sm-B and Sm-B' polypeptides.

12. The method of Claim 11, wherein step (a) comprises
(i) isolating snRNP from cell extracts of human cells by immunoaffinity chromatography using antibodies from serum of a person affected by systemic lupus erythematosus, and
(ii) isolating the naturally-occurring polypeptide from the snRNP.

13. The method of Claim 11, wherein step (f) comprises the steps of
(i) inserting the isolated or synthetic DNA into a host cell to produce a transformed host that is capable of expressing the recombinant polypeptide, and
(ii) propagating the transformed host to produce the recombinant polypeptide.

14. The method of Claim 13, wherein step (i) comprises
inserting the isolated or synthetic DNA into a plasmid to produce a vector capable of transforming a host cell, and transforming the host cell with the vector.

15. A method of producing the recombinant polypeptide of Claim 1 comprising the step of culturing the transformed host of Claim 10.

16. A method of testing a serum sample of a living being to detect whether or not the living being is affected by systemic lupus erythematosus, including the steps of
(a) reacting the recombinant polypeptide of Claim 1 with the serum sample to be tested, and
(b) determining the presence or amount of antibody contained in the sample reactive with the polypeptide.

17. The method of Claim 16, wherein in step (b) the presence or amount of antibody is determined by reacting the mixture with an anti-immunoglobulin antibody covalently attached to an enzyme indicator.

18. The method of Claim 17, wherein the enzyme indicator is lactoperoxidase/alkaline phosphatase.

19. The method of any of Claims 16 to 18, wherein in step (a) the recombinant polypeptide is absorbed on to a test surface.

20. A test kit for the detection of antibodies associated with systemic lupus erythematosus comprising
(a) the recombinant polypeptide of Claim 1,
(b) an antihuman immunoglobulin antibody that is linked to an enzyme, and
(c) a substrate that is capable of reacting with the enzyme to produce a detectable reaction.

21. The test kit of Claim 20, wherein the enzyme is lactoperoxidase or alkaline phosphatase.

## Patentansprüche

1. Ein rekombinantes Polypeptid, umfassend die folgende Sequenz:
val gly lys ser ser lys met leu gln his ile asp tyr arg met arg cys ile leu gln asp
worin das rekombinante Polypeptid mindestens ein Epitop umfaßt, das mit einem Anti-Sm-Antikörper reagieren kann.

2. Ein DNA-Fragment, umfassend eine Nukleotidsequenz, die für das rekombinante Polypeptid von Anspruch 1 codiert.

3. Das DNA-Fragment von Anspruch 2, weiterhin umfassend eine Nukleotidsequenz, die die Expression der Sequenz, die für das rekombinante Polypeptid codiert, beeinflussen kann.

4. Das DNA-Fragment nach Anspruch 3, worin die weitere Nukleotidsequenz für einen Promotor codiert.

5. Das DNA-Fragment nach einem der Ansprüche 2 bis 4, worin das Fragment das Polypeptid von Anspruch 1 in einer Wirtszelle exprimieren kann.

6. Ein Vektor, umfassend das DNA-Fragment nach einem der Ansprüche 2 bis 5.

7. Der Vektor nach Anspruch 6, worin der Vektor eine Wirtszelle transformieren kann.

8. Ein transformierter Wirt, umfassend das DNA-Fragment nach einem der Ansprüche 2 bis 5.

9. Ein transformierter Wirt, umfassend den Vektor nach Anspruch 7.

10. Der transformierte Wirt von Anspruch 9, worin der Wirt das in dem Vektor enthaltene DNA-Fragment exprimieren kann.

11. Ein Verfahren zum Herstellen des rekombinanten Polypeptids von Anspruch 1, umfassend die Schritte des
(a) Isolierens eines natürlich vorkommenden Polypeptids, das mit Systemischem Lupus Erythematodes assoziiert ist,
(b) Identifizierens der Aminosäuresequenz von mindestens einem Teil des natürlich vorkommenden Polypeptids,
(c) Herstellens einer Nukleinsäureprobe, basierend auf der in Schritt (b) identifizierten Aminosäuresequenz, worin die Nukleinsäureprobe mit mindestens einem Teil der DNA, die für das natürlich vorkommende Polypeptid codiert, hybridisieren kann,
(d) Umsetzens der Nukleinsäureprobe mit einer DNA-Bank,
(e) Isolierens der DNA, die mit der Nukleinsäureprobe reagiert, und
(f) Verwendens der isolierten DNA oder einer synthetischen DNA, um das rekombinante Polypeptid herzustellen, worin die synthetische DNA für das gleiche Polypeptid codieren kann, wie die isolierte DNA,
worin das natürlich vorkommende Polypeptid gewählt wird aus der Gruppe, bestehend aus Sm-B- und und Sm-B'-Polypeptiden.

12. Das Verfahren nach Anspruch 11, worin Schritt (a) umfaßt
(i) Isolieren von snRNP aus Zellextrakten von menschlichen Zellen durch Immunaffinitätschromatographie unter Verwendung von Antikörpern aus Serum einer Person, die von Systemischem Lupus Erythematodes betroffen ist, und
(ii) Isolieren des natürlich vorkommenden Polypeptids von dem snRNP.

13. Verfahren nach Anspruch 11, worin Schritt (f) umfaßt die Schritte des
(i) Einbringens der isolierten oder synthetischen DNA in eine Wirtszelle, um einen transformierten Wirt herzustellen, der das rekombinante Polypeptid exprimieren kann, und
(ii) Propagierens des transformierten Wirts, um das rekombinante Polypeptid herzustellen.

14. Verfahren nach Anspruch 13, worin Schritt (i) umfaßt
Einfügen der isolierten oder synthetischen DNA in ein Plasmid, um einen Vektor herzustellen, der eine Wirtszelle transformieren kann, und Transformieren der Wirtszelle mit dem Vektor.

15. Ein Verfahren zum Herstellen des rekombinanten Polypeptids von Anspruch 1, umfassend den Schritt des Kultivierens des transformierten Wirts von Anspruch 10.

16. Ein Verfahren zum Testen einer Serumanalysenprobe eines Lebewesens, um festzustellen, ob oder ob nicht das Lebewesen von Systemischem Lupus Erythematodes betroffen ist, umfassend die Schritte des
(a) Umsetzens des rekombinanten Polypeptids von Anspruch 1 mit der zu testenden Serumanalysenprobe, und
(b) Ermittelns der Anwesenheit oder Menge von in der Analysenprobe enthaltenem Antikörper, der mit dem Polypeptid reaktionsfähig ist.

17. Das Verfahren von Anspruch 16, worin in Schritt (b) die Anwesenheit oder Menge von Antikörper ermittelt wird durch Umsetzen der Mischung mit einem Anti-Immunglobulin-Antikörper, der kovalent mit einem Enzymindikator verbunden ist.

18. Das Verfahren nach Anspruch 17, worin der Enzymindikator Lactoperoxidase/alkalische Phosphatase ist.

19. Das Verfahren nach einem der Ansprüche 16 bis 18, worin in Schritt (a) das rekombinante Polypeptid auf einer Testoberfläche absorbiert ist.

20. Ein Testkit für den Nachweis von Antikörpern, die mit Systemischem Lupus Erythematodes assoziiert sind, umfassend
(a) das rekombinate Polypeptid von Anspruch 1,
(b) einen Antikörper, der gegen menschliches Immunglobulin gerichtet ist, der mit einem Enzym verbunden ist, und
(c) ein Substrat, das mit dem Enzym reagieren kann, um eine nachweisbare Reaktion zu bilden.

21. Der Testkit nach Anspruch 20, worin das Enzym Lactoperoxidase oder alkalische Phosphatase ist.

## Revendications

1. Polypeptide recombinant comprenant la séquence suivante :
val gly lys ser ser lys met leu gln his ile asp tyr arg met arg cys ile leu gln asp
dans laquelle ledit peptide recombinant comprend au moins un épitope pouvant réagir avec un anticorps anti-Sm.

2. Fragment d'ADN comprenant une séquence nucléotide codant pour le polypeptide recombinant de la revendication 1.

3. Fragment d'ADN de la revendication 2 comprenant en plus une séquence nucléotide capable d'influer sur l'expression de la séquence codant pour le polypeptide recombinant.

4. Fragment d'ADN de la revendication 3, dans lequel l'autre séquence de nucléotide code pour un promoteur.

5. Fragment d'ADN selon l'une quelconque des revendications 2 à 4, dans lequel ledit fragment est capable d'exprimer le polypeptide de la revendication 1 dans une cellule hôte.

6. Vecteur comprenant le fragment d'ADN selon l'une quelconque des revendications 2 à 5.

7. Vecteur de la revendication 6, dans lequel ledit vecteur est capable de transformer une cellule hôte.

8. Hôte transformé comprenant le fragment d'ADN selon l'une quelconque des revendications 2 à 5.

9. Hôte transformé comprenant le vecteur de la revendication 7.

10. Hôte transformé de la revendication 9, dans lequel ledit hôte est capable d'exprimer ledit fragment d'ADN contenu dans ledit vecteur.

11. Procédé de production du polypeptide recombinant de la revendication 1 qui comprend les étapes de
(a) isoler un polypeptide d'origine naturelle qui est associé au lupus erythematosus systémique,
(b) identifier la séquence d'amino-acide d'au moins une partie du polypeptide d'origine naturelle,
(c) produire un échantillon d'acide nucléique basé sur la séquence d'amino-acide identifiée à l'étape (b), l'échantillon d'acide nucléique étant capable de réagir avec au moins une portion de l'ADN qui code pour le polypeptide d'origine naturelle,
(d) faire réagir l'échantillon d'acide nucléique avec un ADN de bibliothèque,
(e) isoler l'ADN qui réagit avec l'échantillon d'acide nucléique, et
(f) utiliser l'ADN isolé ou un ADN synthétique pour produire le polypeptide recombinant, dans lequel ledit ADN synthétique est capable de coder le même polypeptide que l'ADN isolé,
dans lequel ledit polypeptide d'origine naturelle est choisi dans un groupe constitué de polypeptides SM-B et SM-B'.

12. Procédé de la revendication 11, dans lequel l'étape (a) comprend
(i) l'isolement de SnRNP d'extraits de cellule de cellules humaines par chromatographie d'immunoaffinité en utilisant des anticorps de sérum d'une personne affectée de lupus erythematosus, et
(il) l'isolement de polypeptide d'origine naturelle provenant du SnRNP.

13. Procédé de la revendication 11, dans lequel l'étape (f) comprend les étapes de
(i) insertion de l'ADN isolé ou synthétique dans une cellule hôte pour produire un hôte transformé qui peut exprimer le polypeptide recombinant, et
(il) cultiver l'hôte transformé pour produire le polypeptide recombinant.

14. Procédé de la revendication 13, dans lequel l'étape (i) comprend l'insertion de l'ADN isolé ou synthétique dans un plasmide pour produire un vecteur capable de transformer une cellule hôte, et la transformation de la cellule hôte avec le vecteur.

15. Procédé de production du polypeptide recombinant de la revendication 1 qui comprend l'étape de culture de l'hôte transformé de la revendication 10.

16. Procédé de test d'un échantillon de sérum d'un être vivant pour détecter si l'être vivant est affecté ou non de lupus erythematosus qui comprend les étapes de
(a) mise en réaction du polypeptide recombinant de la revendication 1 avec l'échantillon de sérum à tester, et
(b) détermination de la présence ou de la quantité d'anticorps contenu dans l'échantillon réagissant avec le polypeptide.

17. Procédé de la revendication 16, dans lequel à l'étape (b) on détermine la présence ou la quantité d'anticorps en faisant réagir le mélange avec un anticorps anti-immunoglobuline fixé de manière covalente à un indicateur enzymatique.

18. Procédé de la revendication 17 dans lequel l'indicateur enzymatique est la lactoperoxydase/phosphatase alcaline.

19. Procédé selon l'une quelconque des revendications 16 à 18, dans lequel à l'étape (a) le polypeptide recombinant est absorbé sur une surface test.

20. Kit de test pour détecter les anticorps associés au lupus érythematosus qui comprend
(a) le polypeptide recombinant de la revendication 1,
(b) un anticorps immunoglobuline antihumain qui est relié à un enzyme, et
(c) un substrat qui est capable de réagir avec l'enzyme pour produire une réaction détectable.

21. Kit de test de la revendication 20, dans lequel l'enzyme est la lactoperoxydase ou la phosphatase alcaline.
